(19) Europäisches Patentamt
European Patent Office
Office européen des brevets

(11) **EP 2 101 275 A1**

(12) **EUROPEAN PATENT APPLICATION**

(43) Date of publication:
**16.09.2009 Bulletin 2009/38**

(51) Int Cl.:
*G06F 19/00* *(2006.01)*

(21) Application number: **08152512.3**

(22) Date of filing: **10.03.2008**

(84) Designated Contracting States:
**AT BE BG CH CY CZ DE DK EE ES FI FR GB GR HR HU IE IS IT LI LT LU LV MC MT NL NO PL PT RO SE SI SK TR**
Designated Extension States:
**AL BA MK RS**

(71) Applicant: **Koninklijke Philips Electronics N.V.**
**5621 BA Eindhoven (NL)**

(72) Inventor: **The designation of the inventor has not yet been filed**

(74) Representative: **van Iersel, Hannie**
**Philips**
**Intellectual Property & Standards**
**P.O. Box 220**
**5600 AE Eindhoven (NL)**

(54) **Method for polynucleotide design and selection**

(57) This invention pertains in general to the field of designing polynucleotide sequences. More particularly the invention relates e.g. to a method for designing probes for DNA microarray technology or primers for the polymerase chain reaction (PCR). The present invention according to some embodiments has the advantage over the prior art that it is based on experimental data, which allows for higher accuracy and better predictions regarding polynucleotide properties so that no experimental validation of the polynucleotide would be needed.

Fig. 1

**Description**

FIELD OF THE INVENTION

This invention pertains in general to the field of designing polynucleotide sequences. More particularly the invention relates to designing of probes for DNA microarray technology or primers for the polymerase chain reaction (PCR).

BACKGROUND OF THE INVENTION

The DNA microarray technology, well known to a person skilled in the art, possesses immense potential for high-throughput analysis of various genomic conditions including SNPs, methylation patterns, copy number variations etc. Several different approaches for probe design are reported in prior art and an increasing number of bioinformatics tools and methods are becoming available for the purpose of probe design. However, the reported accuracy for these tools and methods is still very low, typically less than 50%, which provides a big challenge as a probe generated with various computational approaches subsequently has to be validated experimentally before it can be designated as good probe.

US 2007/0233398 discloses a polynucleotide microarray probe design based on statistical regression analysis of experimental data. The method of US 2007/0233398 uses a statistical model based on the existing features of the probes. The probes designed according to the abovementioned method needs to be validated experimentally for their efficacy and hybridization potential, before they can be used in practice.

Another technology suffering from the same problems as mentioned above is the Polymerase Chain Reaction (PCR) method. Both of these methods work if the probes are able to hybridize well onto the target DNA sequence. So the features associated with the probes are the same in both cases, except for the fact that probes in the case of PCR are called primers and that design of these primers also includes variables such as distance between forward and reverse primer, length of the amplicon, Melting Temperature ($T_m$) uniformity between the $T_m$ of forward and reverse primer and so on.

Hence an improved method would be advantageous providing increased robustness, accuracy and cost efficiency.

SUMMARY OF THE INVENTION

Accordingly, the present invention preferably seeks to mitigate, alleviate or eliminate one or more of the above-identified deficiencies in the art and disadvantages singly or in any combination and solves at least the above mentioned problems e.g. by providing a method for designing a polynucleotide sequence having a desired set of properties, comprising identifying features of a polynucleotide sequence comprised in a database of polynucleotide sequences, based on experimental data. The method may further comprise ranking the features and determining their relative importance compared to the desired set of properties. Moreover, the method may comprise designing the desired polynucleotide sequence having a desired classification based on a first number of the ranked features and by applying a classifier, being trained on how to classify a polynucleotide sequence based on the ranked features, on the desired polynucleotide sequence.

The present invention according to some embodiments has the advantage over the prior art that it is based on experimental data, which allows for higher accuracy and better predictions regarding polynucleotide properties so that the probability that the probe will work in experimental conditions is increased.

Also, the present invention according to some embodiments correctly ranks probe features according to their relative importance. The present invention thus provides fast, robust and automated design of specific polynucleotide sequences, which is critical in generating reliable results in e.g. DNA microarray technology or PCR.

The present invention according to some embodiments thus provides various new features that play a significant role in determining the overall hybridization efficiency of the probe and determining the quality of the probe.

BRIEF DESCRIPTION OF THE DRAWINGS

These and other aspects, features and advantages of which the invention is capable of will be apparent and elucidated from the following description of embodiments of the present invention, reference being made to the accompanying drawings, in which

Fig. 1 is a flowchart showing a method according to an embodiment; and
Fig. 2 shows a probe with two loops. The number of nucleotides before the first loop starts is designated 'a'. The number of nucleotides between the two loops is designated 'b' and the number of nucleotides after the second loop is designated 'c'. Therefore a+b+c is the number of nucleotides, which are not in the loop.

DESCRIPTION OF EMBODIMENTS

Several embodiments of the present invention will be described in more detail below with reference to the accompanying drawings in order for those skilled in the art to be able to carry out the invention. The invention may, however, be embodied in many different forms and should not be construed as limited to the embodiments set forth herein. Rather, these embodiments are provided so that this disclosure will be thorough and complete, and will fully convey the scope of the invention to those skilled in the art. The embodiments do not limit the invention, but the invention is only limited by the appended patent claims. Furthermore, the terminology used in the detailed description of the particular embodiments illustrated in the accompanying drawings is not intended to be limiting of the invention.

The following description focuses on an embodiment of the present invention applicable to a polynucleotide design and in particular to a probe for use in DNA microarray technology. However, it will be appreciated that the invention is not limited to this application but may be applied to many other polynucleotides including for example primers for use in PCR and probes for use in Southern and/or Northern blotting.

Applications and use of the above described method are various and include exemplary fields such as to predict and design a polynucleotide sequence with a desired set of properties, **characterized in that** the method is based on experimental data considering features of existing polynucleotide sequences and rank the polynucleotide features according to their relative importance for predicting the desired set of properties for the polynucleotide sequence.

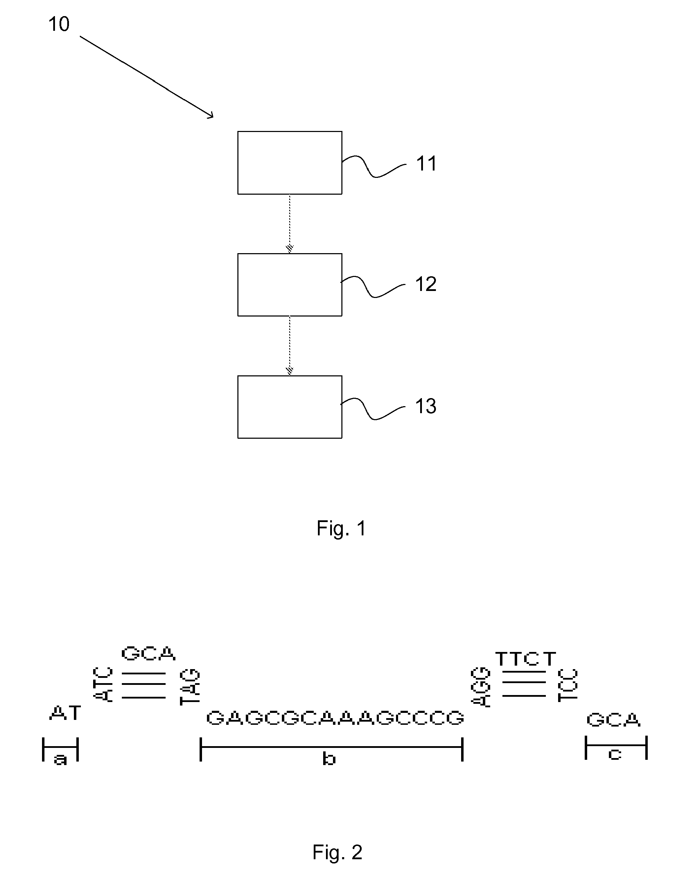

In an embodiment of the invention according to Fig. 1, a method 10 is provided. The method comprises identifying 11 of novel thermodynamics and structural features for a polynucleotide sequence that may be essential for determining the hybridization potential of the probes. These features may be extracted based on experimental data.

Moreover, the method may also comprise ranking 12 of the features based on their relative importance, and classifying the polynucleotide sequences based on these ranked features.

The features are ranked using a feature selection method. They help in determining, which features are important while selecting a good probe using a hybridization model. Higher rank features must be given more importance in the classification model. This approach is important when designing the probes as they increase the probability of hybridization of the probe with the target DNA sequence while performing the experiment.

Existing methods use a limited set of features, which would not be useful for the classification. They also do not rank features.

Furthermore, the method may comprise designing 13 a polynucleotide sequence based on the ranking 12. At present designing of the probe is performed by giving more importance to overall percentage of the nucleotides G and C (GC%), DNA melting temperature ($T_m$) etc.

However, according to an embodiment, it is shown that GC% at 3' end is more important then overall GC%. Similarly for other features a ranking is generated which needs to be taken into account when designing the probes. The main advantage of this method is generation of probes with increased hybridizing efficiency.

Identification of features

A specification of the desired properties of the polynucleotide sequence may be made by identifying the features that are important in hybridization of the probe with target DNA sequence.

From a set of known polynucleotide sequences, feature from e.g. the following categories may be identified:

Feature Category 1. Sequence and composition parameters,
Feature Category 2. Thermodynamic parameters and
Feature Category 3. Secondary structure parameters.

The abovementioned categories may comprise the following methods for extracting probe features:

1. Sequence and composition parameters

The adverse nucleotide base pair composition may be calculated. This may be performed by counting the total number of nucleotides A and T and the total number of nucleotides G and C in the DNA sequence to be analyzed. Another sequence and composition parameter to be considered may be $T_m$ uniformity, which may be calculated according to methods known in the art. DNA melting is the process by which the hydrogen bonds between the strands of the double helix are broken, separating the two strands of DNA. $T_m$ is the temperature at which one half of the DNA duplex will dissociate to become single stranded. $T_m$ indicates the duplex stability. The parameter $T_m$ may also depend on the sequence length as varying the length of the polynucleotide with same composition gives different $T_m$.

A further sequence and composition parameter that may be considered is the GC clamp. The GC clamp method is the number of consecutive G nucleotides and C nucleotides at the 3' end of both the primers or at the 3' end of the probe. It is important for the formation of a complex with the target DNA. Presence of G or C nucleotides within a couple of nucleotides from the 3' end of primers (GC clamp) helps promote specific binding at the 3' end due to the stronger bonding of G and C nucleotides (as G and C form triple hydrogen bonds as opposed to A and T which form double hydrogen bonds). However, higher number of G's or C's should be avoided at 3' as they might lead to false binding. When performing adverse base pair composition calculation, the number of nucleotides A and T may be between about 10 to about 15. The number of nucleotides G and C may be between about 6 to about 9. As an example, there are not more then six A or T nucleotides in a row and the content of the nucleotides G and C is about 40-60% of the total number of nucleotides.

According to one embodiment, the method comprises calculating $T_m$ for sequences less than about 18 nucleotides long, with the fast Wallace method. This method is calculated as follows:

$$T_m = 2(A+T) + 4(G+C) \qquad (1)$$

where A, T, G and C denotes the total prevalence of the respective nucleotide in the sequence. For longer sequences a higher accuracy may be needed. In an embodiment the method may comprise calculating $T_m$ utilizing a Nearest-Neighbor approach as follows:

$$T_m = \frac{\Delta H}{\Delta S + R \ln\left(C_1 - \frac{C_2}{2}\right)} - 273.15°C \qquad (2)$$

where, $\Delta H$ and $\Delta S$ is the standard enthalpy and entropy, $C_1$ and $C_2$ is the initial concentration of single and complementary strand respectively, and R is the universal gas constant.

An optimal product size of an amplicon, i.e. the product of PCR or LCR, may be important to get a proper amplification reaction with e.g. PCR and must be considered when designing a PCR protocol. According to an embodiment, the optimal $T_m$ of an amplicon may be calculated using the formula from Bolton and McCarthy which is:

$$T_m = 81.5 + 16.6(\log_{10}([Na+])) + 0.41*(\%GC) - 600/length \quad (3)$$

where 'Na+' is concentration of sodium ions in the reaction mixture, '%GC' is the content of nucleotides G and C as percent of the total number of nucleotides and 'length' is the total length of the nucleotide sequence.

2. Thermodynamics parameters

Some of the thermodynamics parameters that may play an important role in determining the stability of the polynucleotide probes according to some embodiments are:

- Stacking Energy; dinucleotide base stacking energy represents how easily parts of the DNA de-stack. It is also a measure of DNA unwinding free energy. High energy in base stacking indicates regions of the helix that de-stack or melt more easily; conversely a minimal energy would represent more stable regions.
- Propeller twist; the dinucleotide propeller twist is the value for the flexibility of the helix. Regions with high propeller twist indicate that the helix is quite rigid in that area. Correspondingly, regions that are quite flexible would have low propeller twist values.
- Bendability; sections with high values are more bendable than regions with a low value, which are more flexible. It basically tells the amount of energy required to bend a DNA sequence.
- Duplex Stability Disrupt Energy; regions with a high disrupt energy value, will be more stable than regions with a lower energy value. Stability of the duplex is shown to be dependent on base sequence rather then composition. Therefore increasing the base will increase the amount of energy required to disrupt the duplex.

- Duplex Stability Free Energy; regions with low free energy content will be more stable than regions with high thermodynamic energy content.
- DNA denaturation; DNA regions with a low value are more likely to denature than regions with a higher value. Regions with alternating purines/ pyrimidines steps and A:T rich regions melt more readily. Lower denaturation energy probably due to weak bonding between them makes them easy to denature.
- DNA Bending stiffness; high values correspond to DNA regions that are more rigid, while low values correspond to regions that will bend more easily.
- Internal stability of the polynucleotide as it determines the ability of the polynucleotide to form secondary structure.
- Duplex formation is the formation of secondary structure and any kind of duplex formation will reduce the hybridization potential.
- $\Delta G$, where G is the Gibbs free energy, well known in the art. The stability of hairpin is commonly represented by its $\Delta G$ value, the energy required to break the secondary structure. Larger negative value for $\Delta G$ indicates stable, undesirable hairpins. Presence of hairpins at the 3' end most adversely affects the reaction.

The abovementioned list is not exclusive, and other methods may also be used in the method according to some embodiments.

Regarding internal stability of a polynucleotide, stable 5' termini and unstable 3' termini of primers are used to improve results by reducing false priming on unknown targets. According to one embodiment, this is done while designing the primers by taking into account the various thermodynamics parameters mentioned previously. Furthermore, duplex formation of polynucleotides that may initiate DNA synthesis in case of PCR, may be prevented by low 3' stability, since then a 5' end must also pair in order to form a stable duplex. According to an embodiment, terminal $\Delta G$ is about 8.5 kcal/mol.

3. Secondary structure parameters

Secondary structure formation is considered one of the biggest problems associated with any kind of hybridization. Hairpin loop formation is a secondary structure that may be considered when designing polynucleotides.

Hairpin features may be computed as the number of nucleotides in a polynucleotide sequence, which do form a loop formed by trimers (a so-called SS feature 1) or quadrimers (SS feature 2), or the length of the longest sequence with the loops formed by trimers (SS feature 3) or quadrimers (SS feature 4).

Fig. 2 shows an example of a polynucleotide with two loops formed by trimers. One feature value for this probe (SS feature 1) may be computed as a+b+c, where a is the number of nucleotides before the first loop, b is the number of nucleotides between the loops and c is the number of nucleotides after the second loop. Another feature value for this probe (SS feature 3) may be the maximal value of a, b or c, i.e. max(a;b;c), where a, b and c are denoted as above.

- Another important possible secondary structure that may be considered when extracting features from polynucleotides may be the dimer/cross-dimer formation. When polynucleotides form intermolecular dimers much more readily than hybridizing to target DNA, they reduce the product yield of the reaction of interest.
- Furthermore, the possibility of palindromic sequence (a measure of polynucleotide self-complementarity) may be assessed.
- When features, e.g. the features described above, are specified, the method according some embodiments may further involve creating a vector comprising feature elements, wherein each feature element is normalized, resulting in a long feature vector. The method may then provide for classifying or ranking of the probes based on the vector.
- In an embodiment, only a 3' end hairpin with a $\Delta G$ of -2 kcal/mol and an internal hairpin with a $\Delta G$ of -3 kcal/mol is tolerated.

Also, only a 3' end self-dimer with a $\Delta G$ of -5 kcal/mol and an internal self-dimer with a $\Delta G$ of -6 kcal/mol is tolerated.

Furthermore, a palindrome score of less than 7 is desirable. Palindrome score of a DNA sequence is the maximum number base pair matches that could occur between the 5'-3' sequence and the 3'-5' sequence, divided by two.

In a practical implementation a total of 198 features were identified (11) according to the above embodiments and analyzed in various regions of probes, including the 3' end, 5' end and in the middle of the probe. These features were extracted from the probe sequences that were experimentally proven to be good or bad.

Ranking and classification

The invention may thus comprise identifying the abovementioned features, as well as several other features from experimentally validated probes and rank them. A classification method, also called a classifier, based on the identified and ranked features may be compared to the probe features in order to discriminate probes that perform well during hybridization, so called "good" probes and probes that do not perform well (i.e. give low intensity signal while the target is abundant), so called "bad" probes, using a pattern classification method such as e.g. Support Vector Machines (SVM).

A desired hybridization classification algorithm may extract the most relevant features and eliminate the irrelevant and redundant ones. This is important, since discarding irrelevant features reduces the risk of over fitting, decreases computational complexity and increases overall accuracy. Selection of an optimal subset of features may be carried out using an appropriately designed performance measure to evaluate the ability of the features, and to classify the samples.

In a more specific embodiment of the method the above-mentioned steps 11 and 12 may be performed according to the following, based on an experimental data set. The features may be identified 11 e.g. from 500 different microarray experiments examined by experts and containing more than 80,000 values, as have been confirmed through experiments. An object of this embodiment is to determine sequence composition characteristics for probe sequences that performed well during the hybridization process. Emanating from the DNA sequence, features such as for example primary structure, thermodynamic properties and secondary structure may be identified with a feature extraction methodology. The probes may then be classified as good or bad. The most relevant features are then selected from all the features by feature selection techniques. These features may also be ranked 12 using feature selection techniques.

According to one embodiment, the method comprises ranking the above-mentioned identified features (11) using the Support Vector Machine - Recursive Feature Elimination (SVM-RFE) algorithm.

Briefly, the SVM-RFE takes all the features for training a SVM and then assesses the relative importance of the features in the classifier by the feature-ranking criterion. A SVM preserves the higher rank features and eliminates the lower rank features. The process is repeated (using only survived features) until all the features are assessed. At the end, ranks of all the features are obtained. The feature-ranking criterion for SVM-RFE is:

$$W_r = \sum_{x_i \in S} y_i \lambda_i K(x_i, x) \quad (4)$$

where $\lambda_i$ is the Lagrange multiplier, K(:) is the kernel of the SVM and x is the input data. In one embodiment, a linear kernel for was used for feature selection. An optimal set of features from this ranked list of features is then decided, where the different numbers of top-ranked features is selected to form a series of different feature subsets. The optimal set of features is decided by computing the performance of the classifier with these selected features, iteratively.

Table 1 discloses the feature ranking (12) obtained using SVM-RFE. The table list the top features (ranked) from the abovementioned 198 features divided into the feature categories sequence (monomers, dimers and $T_m$, plus trimers), thermodynamics and secondary structure, obtained according to the above. Overall ranking list of different features are also provided.

**Table 1:** List of top features (ranked) divided into the feature categories sequence, thermodynamics and secondary structure, obtained using SVM-RFE.

| Rank | Type of feature | | | | |
|---|---|---|---|---|---|
| | Monomers, Dimers and $T_m$ | Trimers | Thermodynamics | Secondary Structure | Overall |
| 1 | CG at 3'end | CTG | Stacking | SS Feature 1 | SS Feature 1 |
| 2 | TG | CCG | Propeller twist | SS Feature 3 | CTG |
| 3 | TT in middle | TGC | B-DNA twist | | Bendability |
| 4 | CC in middle | ACT | Bendability | | CG at 3'end |
| 5 | GG in middle | GAG | Entropy | | TCC |
| 6 | GT in middle | AGG | Enthalpy | | CTC |
| 7 | GA at 3'end | TGG | Free Energy | | TGG |
| 8 | $T_m$ | CTC | | | AGG |
| 9 | AC at 5'end | GGT | | | GCC |

(continued)

| Rank | Type of feature | | | | |
| | Monomers, Dimers and $T_m$ | Trimers | Thermodynamics | Secondary Structure | Overall |
|---|---|---|---|---|---|
| 10 | AG at 5'end | TCC | | | $T_m$ |

According to another embodiment, the new feature selection algorithm using nearest neighbor (NN) classifier is used. This method is called the Nearest Neighbor Feature Elimination (NN-FE) method.

The NN-FE method works by dividing the set of features obtained from the above mentioned feature extraction method (11) into two parts, the training set and the validation set. The validation set is subsequently classed using a NN classifier. The ranking (12) may be performed by weighing the features based on misclassifications. For each feature, the weights (measures of classification error) may be computed using the samples, which are misclassified as:

$$W_r = \sum_{i=1}^{N} | x_{ir} - y_{jr} |$$

where $W_r$ is the weight given to feature r, $x_{ir}$ represents the misclassified sample i and feature r from the validation set. The variable $y_{jr}$ represents sample j from the training set, which is closest to sample i and N is the number of samples in the validation set. These weights are used as ranking criterion for the feature. Features with higher weights are given lower rank, because these features are mainly responsible for the misclassification. The top ranked features, with superior accuracy in the validation sets, are selected for the final classification model.

Table 2 discloses the feature ranking (12) obtained using NN-FE. The table list the top features (ranked) from the abovementioned 198 features divided into the feature categories sequence (monomers, dimers and $T_m$, plus trimers), thermodynamics and secondary structure, obtained according to the above. Overall ranking list of different features are also provided.

**Table 2:** List of top features (ranked) divided into the feature categories sequence, thermodynamics and secondary structure, obtained using NN-FE.

| Rank | Type of feature | | | | |
| | Monomers, Dimers and $T_m$ | Trimers | Thermodynamics | Secondary Structure | Overall |
|---|---|---|---|---|---|
| 1 | AA at 5'end | TCA | Bendability | SS Feature 3 | SS Feature 3 |
| 2 | AC at 5'end | CAA | B-DNA twist | SS Feature 1 | AC at 5'end |
| 3 | AC in middle | ATA | Propeller twist | | GAT |
| 4 | GA in middle | GTC | Entropy | | GC in middle |
| 5 | AC in 3' end | ATC | Enthalpy | | TTA |
| 6 | CG at 3'end | AAG | Stacking | | SS Feature 1 |
| 7 | $T_m$ | TAC | Free Energy | | AT at 3'end |
| 8 | AG at 5'end | TTA | | | CAA |
| 9 | GC in middle | TAA | | | GG at 5'end |
| 10 | AT at 3'end | AGA | | | TC at 5'end |

From the tables 1 and 2, three main conclusions may be made. First, it may be observed that the $T_m$ and percentage of content of nucleotides G and C play a significant role in determining the overall hybridization efficiency of a probe. Here, the most important sequence feature is the dimer and trimer fraction of G and C in the middle of, and at 3' end of the probe. The importance of these parameters is well known in the art. However, the model according to an embodiment determines that the secondary structure is a more important factor in determining the level of hybridization efficiency. Secondly, unlike most of prior art, which only highlights the significance of free energy on hybridization, this embodiment shows that the less studied thermodynamic parameters, comprising the DNA twist, stacking energy, propeller twist, bendability, etc. are relatively important in determined the quality of a probe. Finally, in case of secondary structure formation, prior art analyzes only the free energy of the probe. However, this embodiment also takes into account the nucleotide bases that are not involved in loop formation. Interestingly, the analysis according to this embodiment revealed that these base pairs are more significant in determining the stability of hybridization compared to only free energy parameter.

When considering the relative importance of various groups of features in determining the hybridization efficiency of the probe, studying the data from the above-mentioned embodiments, it is observed that the most important parameter is secondary structure formation. These structures are the energy-minimized state of polynucleotide folds, thereby, making thermodynamics another important set of features for hybridization. Furthermore, different types of features and classifiers are analyzed. The results can be seen in table 3. Column 1 of table 3 shows the different types of features. Column 2 shows the size of the set of features for each category listed in Column 1. Column 3 shows the results obtained using different types of classifiers and feature selection methods. According to an embodiment, SVM, k-NN and Fuzzy k-NN are used for classification and SVM-RFE and NN-RFE for feature selection. In all the abovementioned embodiments it is correct SVM with a polynomial kernel of degree 3 is used and k = 19 has been found as an optimal value for the k-NN and Fuzzy k-NN classifiers.

**Table 3:** Classification results using various feature extraction, selection and classification methods.

| | | Classification Accuracy(%) | | | | | |
|---|---|---|---|---|---|---|---|
| | | SVM | | k-NN | | Fuzzy k-NN | |
| Type of feature | Size | RFE | NN-FE | RFE | NN-FE | RPE | NN-FE |
| Sequence and $T_m$ | 81 | 54.3 | 56.5 | 59.5 | 56.8 | 59.0 | 56.8 |
| Sequence (Trimer) | 64 | 61.8 | 58.7 | 59.3 | 58.7 | 58.7 | 57.3 |
| Thermodynamies | 49 | 58.0 | 58.0 | 58.8 | 57.8 | 59.2 | 57.3 |
| Secondary structure | 4 | 49.0 | 49.0 | 51.8 | 51.8 | 55.5 | 53.2 |
| Overall | 198 | 63.5 | 60.7 | 61.3 | 61.3 | 60.8 | 59.8 |

From table 3, it may be observed that the classification accuracy obtained using SVM-RFE for feature selection and SVM for classification is higher compared to other methods. The main observations from table 3 are that classification accuracy achieved by SVM-RFE is 63.5 %, which is higher then any other methods used. This involved combining all the features and selecting the best set of features.

Another important observation is that when different classification methods were used on the results obtained from RFE, different feature groups showed highest significance in different classification methods. In the SVM-RFE classification model, sequence parameters were found to be relatively more important then other feature groups. In case of k-NN-RFE, which also performed equally well, the feature, which proved to be most important was sequence and composition, in particular the $T_m$ of the obtained probes. Similarly, thermodynamics was the most significant feature when the classification was carried out using Fuzzy k-NN-RFE.

However, sequence parameters were the most important when the Nearest-neighbor (RF) method was used for classification. Regarding sequences, the feature that contributed the most was trimers at various positions in the probe.

Design of polynucleotide sequence

According to one embodiment, in order to design a polynucleotide sequence (13), higher ranked features as obtained from hybridization model are given more importance. According to another embodiment, the classification tool is made faster, by picking the top 20 ranking features. Still, similar performance is obtained, as when using all the available features.

In an embodiment, a method 10 is provided for designing a desired polynucleotide sequence having a desired set of properties, comprising identifying 11 features of a polynucleotide sequence comprised in a database of polynucleotide sequences, based on experimental data, ranking 12 the features and determining their relative importance compared to the desired set of properties, and designing 13 the desired polynucleotide sequence having a desired classification, e.g. good or bad, based on a first number of the ranked features and by applying a classifier (SVM, k-NN, fuzzy k-NN), being trained on how to classify a polynucleotide sequence based on the ranked features, on the desired polynucleotide sequence.

The first number may be chosen based upon how many of the ranked features that are to be considered in the design process, e.g. the 10 highest ranked features. For example, this could mean that even if the classifier has been trained based on all of the ranked features, only 10 of the ranked features having the highest rank will be used during classification of the probe.

According to an embodiment the designing is performed by applying the classifier on a polynucleotide sequence, controlling whether the polynucleotide sequence has the desired set of properties based on applying the classifier, and constructing the desired polynucleotide sequence based on the controlled polynucleotide having the desired set of properties.

According to one embodiment of the invention, a method 10 according to above is disclosed, wherein the feature identification 11 method is the Recursive Feature Elimination (RFE) algorithm and/or the Nearest Neighbor - Feature Elimination (NN-FE) method.

In another embodiment the identifying 11 is performed by utilizing a Nearest Neighbor - Feature Elimination (NN-FE) method.

In another embodiment the identifying 11 is performed by utilizing a Recursive Feature Elimination (RFE) algorithm.

Another embodiment discloses a method 10 according to any one of the above embodiments, wherein the ranking 12 of the features is performed by utilizing a Support Vector Machine (SVM) algorithm and nearest neighbor (NN).

Yet another embodiment discloses method 10 according to any one of the embodiments mentioned above, wherein the ranking 12 of the features is performed by the SVM algorithm in conjunction with the RFE algorithm (SVM-RFE).

According to one embodiment, the first number of the ranked features comprises the highest ranked features obtained by the abovementioned SVM-RFE algorithm and are chosen from the group comprising secondary structure feature 1 (SS feature 1), nucleotide triplet CTG, bendability, nucleotide dimer CG at the 3' end, nucleotide triplet TCC, nucleotide triplet CTC, nucleotide triplet TGG, nucleotide triplet AGG, nucleotide triplet GCC and $T_m$.

In another embodiment, the first number of the ranked features comprises the highest ranked features obtained by a Nearest Neighbor - Feature Elimination (NN-FE) method and are chosen from the group comprising of secondary structure feature 3 (SS feature 3), nucleotide dimer AC at the 5' end, nucleotide triplet GAT, nucleotide dimer GC in the middle, nucleotide triplet TTA, secondary structure feature 1 (SS feature 1), nucleotide dimmer AT at the 3' end, nucleotide triplet CAA, nucleotide dimer GC at the 5' end and nucleotide dimer TC at the 5' end.

A further embodiment discloses a method (10) according to any of the above-mentioned embodiments, used for probe design for DNA microarray technology. In another embodiment, the DNA microarray technology is used for SNP detection, gene expression analysis, methylation profiling, miRNA profiling, and/or comparative genomic hybridization.

According to yet another embodiment, the DNA microarray technology is configured to facilitate diagnose and/or screen for single or multiple genetic disorders. A further embodiment discloses a method (10) according to the above embodiments, used for selecting primers for PCR.

In another aspect of the invention, use of the method (10) according to any of the above embodiments is disclosed, where the features used for ranking is comprised in computer software.

According to another embodiment, the computer software is used to predict and design a polynucleotide sequence with a desired set of properties.

In a further embodiment, the polynucleotide sequence is a probe for DNA microarray technology.

In another embodiment, the DNA microarray technology is used for SNP detection, gene expression analysis, methylation profiling, miRNA profiling, and/or comparative genomic hybridization.

A further embodiment discloses use according to the above computer software embodiments, wherein the DNA microarray technology is intended to diagnose and/or screen for single or multiple genetic disorders.

Another embodiment discloses use of the computer software according to above, wherein the polynucleotide sequence is a primer for PCR.

The invention may be implemented in any suitable form including hardware, software, firmware or any combination of these. However, preferably, the invention is implemented as computer software running on one or more data processors and/or digital signal processors. The elements and components of an embodiment of the invention may be physically, functionally and logically implemented in any suitable way. Indeed, the functionality may be implemented in a single unit, in a plurality of units or as part of other functional units. As such, the invention may be implemented in a single unit, or may be physically and functionally distributed between different units and processors.

Although the present invention has been described above with reference to specific embodiments, it is not intended to be limited to the specific form set forth herein. Rather, the invention is limited only by the accompanying claims and, other embodiments than the specific above are equally possible within the scope of these appended claims. In the claims, the term "comprises/comprising" does not exclude the presence of other elements or steps. Furthermore, although individually listed, a plurality of means, elements or method steps may be implemented by e.g. a single unit or processor. Additionally, although individual features may be included in different claims, these may possibly advantageously be combined, and the inclusion in different claims does not imply that a combination of features is not feasible and/or advantageous. In addition, singular references do not exclude a plurality. The terms "a", "an", "first", "second" etc do not preclude a plurality. Reference signs in the claims are provided merely as a clarifying example and shall not be construed as limiting the scope of the claims in any way.

**Claims**

1. A method (10) for designing a desired polynucleotide sequence having a desired set of properties, comprising

   - identifying (11) features of a polynucleotide sequence comprised in a database of polynucleotide sequences, based on experimental data,
   ranking (12) said features and determining their relative importance compared to the desired set of properties,
   - designing (13) said desired polynucleotide sequence having a desired classification based on a first number of said ranked features and by applying a classifier (SVM, k-NN, fuzzy k-NN), being trained on how to classify a polynucleotide sequence based on said ranked features, on said desired polynucleotide sequence.

2. The method according to claim 1, wherein said designing is performed by

   - applying said classifier on a polynucleotide sequence,
   - controlling whether said polynucleotide sequence has the desired set of properties based on applying said classifier,
   - constructing said desired polynucleotide sequence based on said controlled polynucleotide having the desired set of properties.

3. The method according to claim 1 or 2, wherein said identifying (11) is performed by utilizing a Nearest Neighbor - Feature Elimination (NN-FE) method.

4. The method according to claim 1 or 2, wherein said identifying (11) is performed by utilizing a Recursive Feature Elimination (RFE) algorithm.

5. The method according to claims 1, 2, 3 or 4, wherein said ranking (12) of said features is performed by utilizing a Support Vector Machine (SVM) algorithm and nearest neighbor (NN).

6. The method according to claims 1, 2, 3 or 4, wherein said ranking (12) of said features is performed by utilizing a SVM algorithm in conjunction with a RFE algorithm (SVM-RFE).

7. The method according to claim 6, wherein the first number of said ranked features comprises features with highest rank obtained by a SVM-RFE algorithm and are chosen from the group comprising secondary structure feature 1 (SS feature 1), nucleotide triplet CTG, bendability, nucleotide dimer CG at the 3' end, nucleotide triplet TCC, nucleotide triplet CTC, nucleotide triplet TGG, nucleotide triplet AGG, nucleotide triplet GCC and $T_m$.

8. The method according to claims 1, 2 or 3, wherein the first number of said ranked features comprises features with highest rank obtained by a Nearest Neighbor-Feature Elimination (NN-FE) method and are chosen from the group comprising secondary structure feature 3 (SS feature 3), nucleotide dimer AC at the 5' end, nucleotide triplet GAT, nucleotide dimer GC in the middle, nucleotide triplet TTA, secondary structure feature 1 (SS feature 1), nucleotide dimmer AT at the 3' end, nucleotide triplet CAA, nucleotide dimer GC at the 5' end and nucleotide dimer TC at the 5' end.

9. The method according to claim 1, wherein said first number is any number between 0 and the total number of said features.

10. The method according to claim 1, 7 or 8 used for probe design for DNA microarray technology.

11. The method according to claim 1, comprising selecting primers for polymerase chain reaction (PCR).

12. Use of the method according to claim 1, wherein said features used for ranking is comprised in computer software.

Fig. 1

Fig. 2

Europäisches
Patentamt

European
Patent Office

Office européen
des brevets

# EUROPEAN SEARCH REPORT

Application Number

EP 08 15 2512

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | HU GUANGAN ET AL: "Selection of long oligonucleotides for gene expression microarrays using weighted rank-sum strategy" BMC BIOINFORMATICS, vol. 8, September 2007 (2007-09), pages 350-1-350-13, XP021031497 ISSN: 1471-2105 * abstract * * pages 350-2 - pages 350-3 * * pages 350-6, right-hand column, paragraph 2 - pages 350-7, right-hand column, last paragraph * * pages 350-9, right-hand column, last paragraph - pages 350-12, left-hand column, last paragraph * * figures 1,2 * | 1-12 | INV. G06F19/00 |
| X | US 2003/096986 A1 (MEI RUI [US] ET AL) 22 May 2003 (2003-05-22) * abstract * * paragraph [0070] - paragraph [0081] * * paragraph [0092] - paragraph [0100] * * figures 3,10,13 * | 1-12 | |

TECHNICAL FIELDS SEARCHED (IPC)

G06F

-/--

The present search report has been drawn up for all claims

| Place of search | Date of completion of the search | Examiner |
|---|---|---|
| Munich | 13 November 2008 | Hilbig, Matthias |

EPO FORM 1503 03.82 (P04C01)

| | Europäisches Patentamt<br>European Patent Office<br>Office européen des brevets | **EUROPEAN SEARCH REPORT** | **Application Number**<br>EP 08 15 2512 |

## DOCUMENTS CONSIDERED TO BE RELEVANT

| Category | Citation of document with indication, where appropriate,<br>of relevant passages | Relevant<br>to claim | CLASSIFICATION OF THE<br>APPLICATION (IPC) |
|---|---|---|---|
| X | TOLSTRUP N ET AL: "OligoDesign: optimal design of LNA (locked nucleic acid) oligonucleotide capture probes for gene expression profiling"<br>NUCLEIC ACIDS RESEARCH, OXFORD UNIVERSITY PRESS, SURREY, GB,<br>vol. 31, no. 13,<br>1 January 2003 (2003-01-01), pages 3758-3762, XP002285001<br>ISSN: 0305-1048<br>* abstract *<br>* page 3759, left-hand column, paragraph 1 - page 3760, left-hand column, paragraph 1 * | 1-12 | |
| D,X | US 2007/233398 A1 (SHCHEGROVA SVETLANA V [US] ET AL) 4 October 2007 (2007-10-04)<br>* abstract *<br>* paragraph [0038] - paragraph [0063] *<br>* figures 1-4 *<br>* claim 1 * | 1-12 | |
| | | | **TECHNICAL FIELDS<br>SEARCHED (IPC)** |

The present search report has been drawn up for all claims

| Place of search | Date of completion of the search | Examiner |
|---|---|---|
| Munich | 13 November 2008 | Hilbig, Matthias |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 08 15 2512

This annex lists the patent family members relating to the patent documents cited in the above-mentioned European search report.
The members are as contained in the European Patent Office EDP file on
The European Patent Office is in no way liable for these particulars which are merely given for the purpose of information.

13-11-2008

| Patent document cited in search report | Publication date | Patent family member(s) | Publication date |
|---|---|---|---|
| US 2003096986 A1 | 22-05-2003 | US 2003130802 A1 | 10-07-2003 |
| US 2007233398 A1 | 04-10-2007 | NONE | |

EPO FORM P0459

For more details about this annex : see Official Journal of the European Patent Office, No. 12/82

**EP 2 101 275 A1**

**Patent documents cited in the description**

- US 20070233398 A